# EUROPÄISCHE PATENTSCHRIFT

(11) **EP 3 742 956 B1**
(45) Veröffentlichungstag und Bekanntmachung des Hinweises auf die Patenterteilung: **28.12.2022**
(21) Anmeldenummer: 19701842.7
(22) Anmeldetag: 25.01.2019
(51) Int. Cl.: A61B 3/10, G01B 9/02, G01B 9/02015, G01B 9/02091

(54) **VERFAHREN ZUR ERZEUGUNG EINES ZWEIDIMENSIONALEN INTERFEROGRAMMS MIT EINEM FREISTRAHL-INTERFEROMETER DES MICHELSON-TYPS**
METHOD FOR GENERATING A TWO-DIMENSIONAL INTERFEROGRAM USING A MICHELSON-TYPE FREE BEAM INTERFEROMETER
PROCÉDÉ DE PRODUCTION D'UN INTERFÉROGRAMME BIDIMENSIONNEL AU MOYEN D'UN INTERFÉROMÈTRE À FAISCEAU LIBRE DE TYPE MICHELSON

(30) Priorität: 26.01.2018 DE 102018101768
(43) Veröffentlichungstag der Anmeldung: 02.12.2020
(73) Patentinhaber: Visotec GmbH, 23552 Lübeck (DE)
(72) Erfinder: MÜNST, Michael, 23562 Lübeck (DE); SUDKAMP, Helge, 23552 Lübeck (DE); KOCH, Peter, 23558 Lübeck (DE); HÜTTMANN, Gereon, 23564 Lübeck (DE)
(74) Vertreter: RCD Patent Giesen, Schmelcher & Griebel Patentwanwälte PartG mbB
(86) Internationale Anmeldenummer: PCT/EP2019/051816
(87) Internationale Veröffentlichungsnummer: WO 2019/145459

(56) Entgegenhaltungen:
- WO-A1-2017/029160
- WO-A2-2009/111609
- HELGE SUDKAMP ET AL: "In-vivo retinal imaging with off-axis full-field time-domain optical coherence tomography", OPTICS LETTERS, Bd. 41, Nr. 21, 1. November 2016 (2016-11-01), Seite 4987, XP055562783, US ISSN: 0146-9592, DOI: 10.1364/OL.41.004987

## Beschreibung

Die Erfindung betrifft ein Verfahren zur Erzeugung eines zweidimensionalen Interferogramms mit einem Freistrahl-Interferometer des Michelson-Typs umfassend eine ausgedehnte, räumlich teilkohärente Lichtquelle und einen zweidimensionalen Lichtdetektor, wobei Licht der Lichtquelle durch einen Strahlteiler mit einem halbdurchlässigen Strahlteilerspiegel in einen Probenlichtstrahl und in einen Referenzlichtstrahl aufgeteilt und einem Probenarm und einem in seiner Länge veränderlichen Referenzarm zugeführt wird.

Unter einer ausgedehnten, räumlich teilkohärenten Lichtquelle ist hier zu verstehen, dass die Lichtquelle eine räumlich teilkohärentes Licht aussendende Fläche aufweist, wobei Lichtmoden, die von einem ersten Flächenrandbereich ausgesendet werden, mit Lichtmoden von einem zweiten Flächenrandbereich, der dem ersten gegenüber liegt, d.h. etwa um einen Flächendurchmesser vom ersten beabstandet ist, nicht mehr interferieren. Ein Beispiel für eine solche Lichtquelle ist eine nicht fasergekoppelte Superlumineszenz-Diode (SLD).

Freistrahl-Interferometer des Michelson-Typs kommen u. a. in Vorrichtungen zur Optischen Kohärenztomographie (OCT) zum Einsatz, die dazu ausgelegt sind, das gesamte von einer flächigen Probe gestreute Licht simultan zu detektieren ("full-field OCT, FF-OCT) und den lateralen Streuorten auf der Probe zuzuordnen. Die auf die Arbeitsgruppe der Erfinder zurückgehende Druckschrift WO 2017/029160 A1 beschreibt beispielsweise eine solche Vorrichtung und ein zugehöriges Messverfahren. Weiterhin sind als Stand der Technik die Druckschriften WO 2009/111609 A2, DE 10 2006 031822 B3 und GB 2 393 263 A zu nennen.

Insbesondere verwenden Verfahren und Vorrichtung der WO 2017/029160 A1 räumlich teilkohärentes Licht mit kurzer Kohärenzlänge - kleiner als 25 Mikrometer - aus dem NIR-Spektrum, und das Freistrahl-Interferometer weist einen durch einen verschiebbaren Referenzspiegel in seiner Länge veränderlichen Referenzarm auf. Zur Interferenz auf dem zweidimensionalen Lichtdetektor - z.B. einer CCD- oder CMOS-Kamera - am Ausgang des Interferometers kommt es nur bei Übereinstimmung der optischen Weglängen von Referenzlicht und gestreutem Probenlicht innerhalb der Kohärenzlänge. Der veränderliche Referenzarm erlaubt somit, Probenlicht aus verschiedenen Probentiefen mit dem Referenzlicht zur Interferenz zu bringen. Die Längenänderung des Referenzarmes kann durch den entlang der optischen Achse verschiebbaren Referenzspiegel nach Art der bekannten "time-domain" (TD) OCT realisiert werden, wobei eine repetierende Bewegung des Spiegels für wiederholte Tiefenscans möglich ist.

Gestreutes Probenlicht, das nicht mit dem Referenzlicht interferiert, gelangt gleichzeitig als Hintergrund auf den Lichtdetektor. Um den Hintergrund vom Interferogramm in einer Nachbearbeitung zu separieren und Phase und Amplitude des Probenlichtes zu bestimmen, werden Referenz- und Probenlicht um einen vorbestimmten Winkel gegeneinander verkippt überlagert und die dann entstehende Intensitätsverteilung detektiert ("off-axis", OA). Das Interferogramm weist dadurch ein zusätzliches Muster von Interferenzstreifen entlang der Detektorfläche auf. Bei der Fourier-Transformation der erfassten Bilddaten liegen die dem Interferogramm zuzuordnenden Fourier-Komponenten dadurch um einen vorbestimmten k-Vektor verschoben gegenüber den Fourier-Koeffizienten des Hintergrundes.

Die Vorrichtung und das Verfahren der WO 2017/029160 A1 sind nach dem zuvor Gesagten durch den Begriff "Off-Axis Full-Field Time-Domain OCT" (OA-FF-TD-OCT) von anderen OCT-Verfahren klar abgegrenzt.

Bei der praktischen Umsetzung der Lehre der WO 2017/029160 A1 insbesondere in Anlehnung an die dortige Fig.1 zeigt sich das Problem, dass ein verkippter Referenzspiegel den Referenzstrahl zwar verkippt auf den Lichtdetektor einstrahlt, aber eben auch gegenüber dem Probenstrahl seitlich versetzt. Dieser laterale Versatz erweist sich bei geringem Bauraum für die optische Anordnung und/oder bei großen Armlängen der Interferometerarme im Vergleich zum Strahldurchmesser als problematisch. Der Detektor wird dann nicht mehr zentral vom Referenzstrahl getroffen.

Wenn das OA-FF-TD-OCT Verfahren zur Untersuchung des Augenhintergrundes verwendet wird, ergibt sich aus technischen Gründen eine Probenarmlänge von einigen 100 mm. Der Referenzarm muss dieselbe optische Länge wie der Probenarm aufweisen. Beispielsweise beträgt ein typischer Abstand des Referenzspiegels zum Lichtdetektor 250 mm. Der Winkel, den Referenz- und Probenstrahl auf dem Lichtdetektor einschließen müssen, um ein abtastbares Streifenmuster zu erzeugen, hängt vom Durchmesser der einzelnen lichtsensitiven Pixel des Detektors, z.B. 3,45 µm (Sony 252 Sensor mit 2045 × 1536 Pixeln), und von der Zentralwellenlänge der räumlich teilkohärenten Lichtquelle, z.B. 830 nm, ab und ergibt sich beispielsweise zu *α* ≈ 3,5 ° . Der laterale Versatz des Referenzlichts gegen das Probenlicht *ΔS* auf dem Bildsensor beträgt Δ*S* = tan *α* × *l_{R}* mit *l_{R}* als Abstand des Referenzspiegels (Mittelpunkt) zum Bildsensor. Mit den vorstehenden Beispielwerten ergibt sich auf dem Lichtdetektor ein lateraler Versatz von 15,26 mm. Der Strahldurchmesser müsste dann mindestens die 15,26 mm zuzüglich der Detektorgröße, ca. 7 mm, besitzen und ein Großteil des Lichtes würde dabei gar nicht genutzt werden. Es verringert sich bei räumlich teilkohärenten Lichtquellen auch der Kontrast des Interferenzsignals und damit Empfindlichkeit und Kontrastumfang der Retinabilder.

Überdies sorgt jede Bewegung des Referenzspiegels zur Veränderung der Referenzarmlänge auch noch für eine weitere Änderung des lateralen Versatzes auf dem Lichtdetektor.

Es ist daher die Aufgabe der Erfindung, ein Verfahren zur Interferometrie mit einem Freistrahl-Interferometer des Michelson-Typs vorzuschlagen, bei dem Proben- und Referenzlicht auf dem Lichtdetektor ohne lateralen Versatz und miteinander einen vorbestimmten Winkel größer als null einschließend überlagert werden können.

Die Aufgabe wird gelöst durch ein Verfahren zur Erzeugung eines zweidimensionalen Interferogramms mit einem Freistrahl-Interferometer des Michelson-Typs umfassend eine ausgedehnte, räumlich teilkohärente Lichtquelle und einen zweidimensionalen Lichtdetektor, wobei Licht der Lichtquelle durch einen Strahlteiler mit einem halbdurchlässigen Strahlteilerspiegel in einen Probenlichtstrahl und in einen Referenzlichtstrahl aufgeteilt und einem Probenarm und einem Referenzarm zugeführt wird, wobei der von einer Probe wiederkehrende Probenlichtstrahl über den Strahlteilerspiegel auf den Lichtdetektor geführt wird, wobei der aus dem Referenzarm austretende Referenzlichtstrahl auf dem Lichtdetektor einen vorbestimmten Winkel größer als null mit dem Probenlichtstrahl einschließt, wobei die Länge des Referenzarms veränderlich ist, dadurch gekennzeichnet, dass der Referenzlichtstrahl mittels einer ungeraden Anzahl von Reflexionen in jeder Reflexionsebene in wenigstens einem Referenzarmabschnitt zu sich selbst seitlich versetzt und entgegengesetzt parallel verlaufend durch ein am Ausgang des Referenzarmes fixiert angeordnetes, mittels Beugung oder Brechung Licht ablenkendes Element geführt wird.

Die Unteransprüche geben vorteilhafte Ausgestaltungen an.

Es ist zunächst festzuhalten, dass die geschilderte Problematik bei anderen Interferometer-Typen, z.B. einem Mach-Zehnder-Interferometer, durchaus leicht vermeidbar ist. Das Michelson-Interferometer besticht jedoch durch seinen einfachen Aufbau und die geringe Anzahl benötigter optischer Komponenten und ist daher für die Serienfertigung von OCT-Systemen besonders attraktiv.

In einem klassischen Michelson-Interferometer wird der Referenzstrahl vom Referenzspiegel in sich selbst reflektiert, und somit fallen Eingang und Ausgang des Referenzarmes zusammen. Erfindungsgemäß sind im vorliegenden Fall Eingang und Ausgang des Referenzarmes räumlich zu separieren, damit am Ausgang ein zusätzliches optisches Element zur Ablenkung des zurückkehrenden Referenzlichts um einen vorbestimmten Winkel erfindungsgemäß fixiert angeordnet werden kann. Da dieser vorbestimmte Winkel nur wenige Grad beträgt, soll das Licht ablenkende Element die Ablenkung mittels Beugung oder Brechung hervorrufen, nicht aber mittels Spiegelung. Das Licht ablenkende Element kann vorzugsweise ein Prisma oder ein Beugungsgitter sein.

Wenn das Referenzlicht im Referenzarm so geführt wird, dass es immer, insbesondere bei jeder beliebigen Länge des Referenzarmes, am gleichen Ort und unter dem gleichen Winkel auf das Licht ablenkende Element trifft, dann kann das erfindungsgemäß fixierte, brechende oder beugende Element so justiert werden, dass das Referenzlicht stets unter dem vorbestimmten Ablenkwinkel und ohne lateralen Versatz zum Probenlicht auf den Detektor gelangt.

Der Fachmann weiß, dass Retroreflektoren den Referenzstrahl so spiegeln, dass er zu sich selbst seitlich versetzt und entgegengesetzt parallel verläuft. Diese Bedingungen lassen sich also mit einem Retroreflektor anstelle des herkömmlichen Referenzspiegels einrichten. In Betracht kommende Retroreflektoren sind hierbei der 90°-Winkelspiegel oder auch der klassische Corner-Cube, also Anordnungen aus zwei oder drei zueinander im 90°-Winkel orientierten Spiegeln.

Erfindungsgemäß soll wenigstens ein Abschnitt des Referenzsarmes einen solchen Verlauf des Referenzlichts aufweisen. Vorzugsweise wird die Länge des Referenzarmes in dem wenigstens einen Referenzarmabschnitt geändert, in dem der Referenzlichtstrahl zu sich selbst seitlich versetzt und entgegengesetzt parallel verläuft. Dann beeinflusst die Referenzarmlänge nicht den Ort und Winkel des Referenzstrahls beim Treffen auf das am Ausgang fixierte Licht ablenkende Element.

Unerwartet stellt sich allerdings heraus, dass ein Retroreflektor für die Umsetzung der Erfindung nicht geeignet ist. Die Interferogramme erweisen sich dann nämlich zu den Bildrändern hin als sehr schwach bis verschwindend.

Die Ursache dafür ist die ausgedehnte, räumlich teilkohärente Lichtquelle.

Verwendet man den klassische Michelson-Aufbau mit Winkel null zwischen Proben- und Referenzlicht, dann erhält man auf allen Pixeln des Lichtdetektors Interferenzen, weil beide Strahlanteile genau zweimal gespiegelt worden sind, wenn sie am Detektor eintreffen, nämlich einmal am Strahlteiler und einmal am Referenzspiegel bzw. an der Probe. Ein Retroreflektor wie der 90°-Winkelspiegel vollführt indes zwei Spiegelungen, und dies hat zur Folge, dass der aus dem Referenzarm austretende Referenzstrahl gegenüber dem Probenstrahl spiegelverkehrt ist. Lichtanteile von Proben- und Referenzlicht, die auf Pixeln an einem Seitenrand des Detektors zusammentreffen, stammen tatsächlich von zwei gegenüberliegenden Flächenrandbereichen der flächig ausgedehnten Lichtquelle und interferieren daher nicht.

Das erkannte Problem kann erfindungsgemäß dadurch behoben werden, dass der Referenzlichtstrahl mittels einer ungeraden Anzahl von Reflexionen in jeder Reflexionsebene in wenigstens einem Referenzarmabschnitt zu sich selbst seitlich versetzt und entgegengesetzt parallel verlaufend geführt wird. Beispielsweise können also drei Reflexionen in einer Reflexionsebene ausgeführt werden, um ein zur Interferenz mit dem Probenlicht geeignetes Referenzlicht zu erhalten.

Als Reflexionsebene wird hier eine Ebene im Raum bezeichnet, die von der Normalenrichtung eines ebenen Spiegels und der Einfalls- oder Ausfallsrichtung eines an dem ebenen Spiegel reflektierten Lichtstrahls aufgespannt wird. Der Aufbau des Interferometers legt alle Strahlrichtungen und Spiegelanordnungen fest, d.h. alle Reflexionsebenen sind aufbau-immanent und vorbekannt. In der Regel wird man für den Weg des Referenzlichts im Referenzarm nur genau eine Reflexionsebene vorsehen. Wenigstens eine wohldefinierte Reflexionsebene ist jedoch zwingend vorhanden, da der Referenzstrahl zwischen Eingang und Ausgang des Referenzarmes seitlich versetzt werden muss.

Ein Retroreflektor nach Art eines Corner-Cube kann zwar drei Spiegelungen durchführen, doch finden diese dann in wenigstens zwei verschiedenen Reflexionsebenen statt. Das austretende Referenzlicht ist auch in diesem Fall nicht zur Interferenz mit dem Probenlicht auf dem gesamten Detektor geeignet.

Führt man indes eine ungerade Anzahl von Reflexionen in jeder Reflexionsebene - so es denn mehr als eine gibt - für das Licht im Referenzarm durch, dann kann das Referenzlicht genauso gespiegelt aus dem Referenzarm austreten, wie es nach der nur einmaligen Spiegelung im herkömmlichen Michelson-Interferometer der Fall wäre. Das zusätzlich erfindungsgemäß fixiert angeordnete, Licht ablenkende Element am Ausgang führt den Referenzstrahl dann unter einem vorbestimmten Winkel gegen den Probenstrahl auf einen vorbestimmten Bereich des Lichtdetektors - üblich die gesamte Detektorfläche. Proben- und Referenzlicht können auf dem gesamten Lichtdetektor interferieren. Beide Strahlen müssen nicht breiter als der Lichtdetektor sein. Eine Änderung der Referenzarmlänge hat keinen Einfluss auf die laterale Position des Referenzstrahls auf dem Detektor.

Das Referenzlicht kann auf seinem Weg im Referenzarm beliebig viele beugende oder brechende optische Komponenten transmittieren und dadurch Änderungen der Strahlrichtungen erfahren. Dies hat keinen weiteren Einfluss auf seine Fähigkeit zur vollständigen Interferenz mit dem Probenlicht, solange es nur zu wenigstens einer Reflexion zwischen Eingang und Ausgang des Referenzarmes kommt.

Die exakte Inversion der Referenzstrahlrichtung mit seitlichem Versatz zwischen dem einlaufenden und auslaufenden Strahl in dem wenigsten einen, bevorzugt zur Änderung der Referenzarmlänge vorgesehenen Referenzarmabschnitt kann auf eine Vielzahl von Arten erreicht werden, die sich nicht alle hier wiedergeben lassen.

Stattdessen sollen Ausführungsbeispiele für Interferometer-Anordnungen, die das erfindungsgemäße Verfahren umsetzen, anhand von Figuren näher erläutert werden. Dabei zeigt:
Fig. 1 ein Ausführungsbeispiel mit drei Spiegeln zur seitlich versetzten Umkehr der Strahlrichtung und mit einem Prisma als Licht ablenkendem Element;
Fig. 2 ein Ausführungsbeispiel mit einem Prisma mit zwei Licht brechenden Flächen und einer spiegelnden Fläche zur seitlich versetzten Umkehr der Strahlrichtung und mit einem Beugungsgitter als Licht ablenkendem Element.

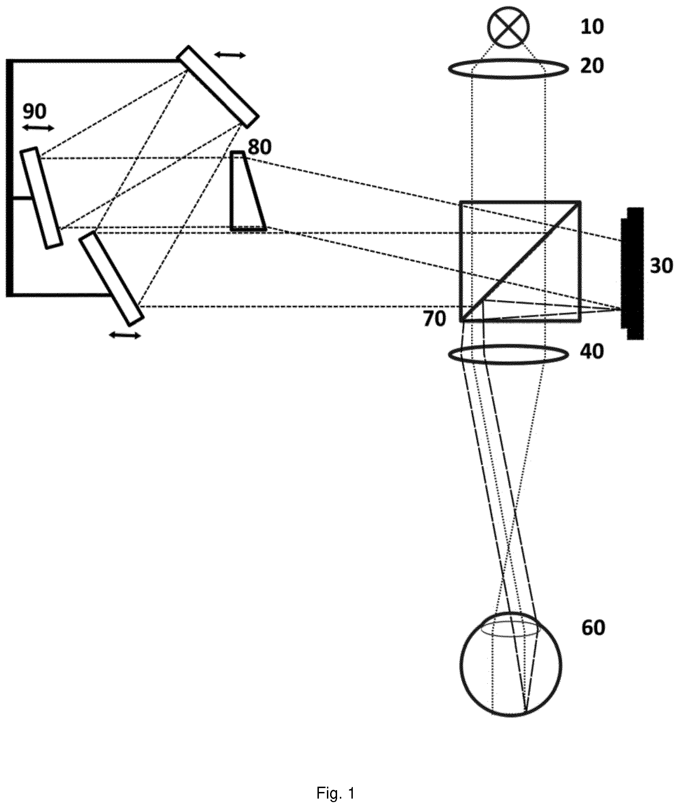

In Fig. 1 ist ein Freistrahl-Interferometer des Michelson-Typs dargestellt, das das erfindungsgemäße Verfahren realisiert. Das Licht einer ausgedehnten, räumlich teilkohärenten Lichtquelle (10) wird zunächst durch eine Kollimationslinse (20) kollimiert und hiernach mit einem Strahlteiler-Würfel (70) in Proben- und Referenzlicht aufgeteilt. Das Probenlicht soll auf die Retina eines lebenden Auges (60) möglichst großflächig eingestrahlt werden. Es wird dazu mit einer Fokussierlinse (40) vor dem Auge (60) fokussiert, damit die biologische Augenlinse die vom Fokus ausgehenden divergenten Strahlen in geeigneter Weise bricht. Das von der Retina gestreute Probenlicht wird über die Augenlinse und die Fokussierlinse (40) unter Spiegelung am Strahlteiler-Würfel (70) auf eine elektronische Kamera (30) abgebildet.

Zugleich wird der Referenzlichtstrahl kollimiert über eine Anordnung (90) von drei Spiegeln in einer einzigen, den Spiegeln gemeinsamen Reflexionsebene so geführt, dass er zu sich selbst seitlich versetzt und entgegengesetzt parallel verläuft. Er trifft dann auf ein Prisma (80), das den Strahl bricht und unter einem vorbestimmten Winkel gegenüber dem Probenlichtstrahl auf den zweidimensionalen Lichtdetektor (30) lenkt. Das Prisma (80) ist dabei fixiert angeordnet, während die Anordnung (90) der drei Spiegel entlang der Richtung der Einstrahlung bzw. Ausstrahlung des Referenzlichtstrahls verschiebbar ausgebildet ist. Dabei deuten in Fig. 1 gezeichnete Verbindungslinien der einzelnen Spiegel zu einem Balken eine gemeinsame Halterung der Spiegel an, die dafür sorgt, dass die Spiegel ihre Anordnung zueinander auch während der Verschiebung beibehalten. Diese Verschiebung der Anordnung (90) ändert zwar die Länge des Referenzarmes, nicht aber den Winkel und Ort des Auftreffens des Referenzlichts auf dem Lichtdetektor (30).

Aus den in Fig. 1 dargestellten Strahlverläufen ist auch ersichtlich, dass Lichtmoden, die hinter der Kollimationslinse (20) beispielsweise im linken Randbereich des Lichtstrahls propagieren, sowohl vom Probenarm als auch vom Referenzarm in den unteren Randbereich des Detektors (30) geführt werden. Würde sich anstelle der Anordnung (90) ein 90°-Winkelspiegel als Retroreflektor im Referenzarm befinden, träfen diese Lichtmoden stattdessen aus dem Probenarm im unteren und aus dem Referenzarm im oberen Bereich des Detektors (30) ein.

Es ist einzusehen, dass man sich eine Vielzahl von Spiegelanordnungen auch mit mehr als drei Spiegeln überlegen kann, in denen die Normalen aller Spiegel in einer gemeinsamen Ebene liegen, wobei die Lichteinstrahlung entlang dieser Ebene zu einem seitlich versetzten und entgegengesetzt parallel verlaufenden Lichtstrahl führt. Doch nur jene Varianten, bei denen der Lichtstrahl insgesamt eine ungerade Anzahl von Reflexionen erfährt, sind erfindungsgemäß zweckdienlich für die Interferometrie mit einer ausgedehnten, räumlich teilkohärenten Lichtquelle.

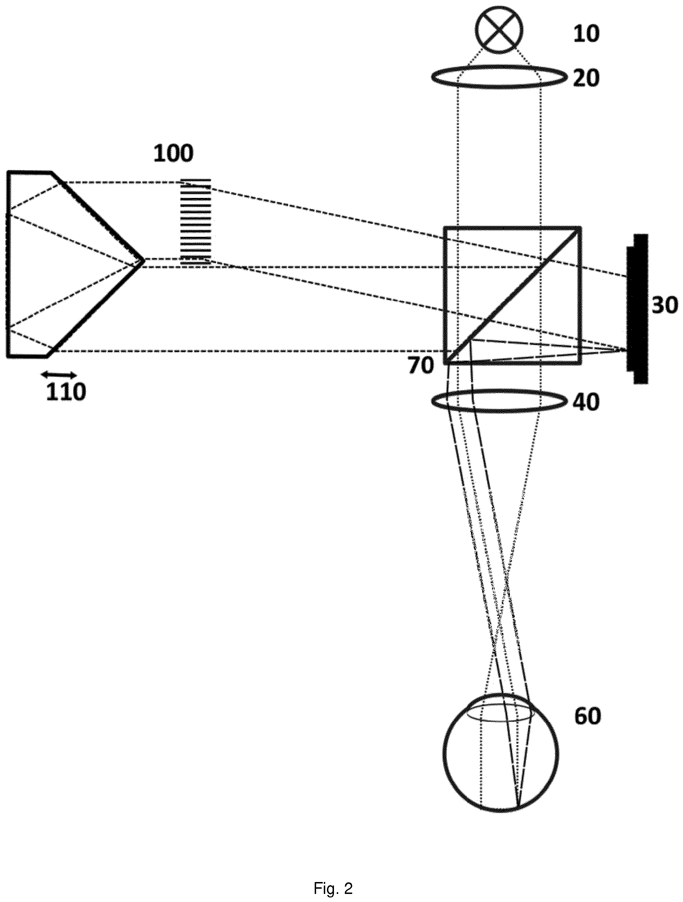

In Fig. 2 ist eine zweite, recht einfache und daher bevorzugte Ausgestaltung des Referenzarmes dargestellt, die das erfindungsgemäße Verfahren umsetzt. Die übrigen Komponenten des Freistrahl-Interferometers sind dieselben wie in Fig. 1.

Der seitlich versetzte und parallel entgegengesetzte Verlauf des Referenzlichtstrahls kann auch mit nur genau einem Spiegel erreicht werden, wobei aber das Licht nicht senkrecht auf den Spiegel einfällt. Der Referenzlichtstrahl wird beispielsweise in Fig. 2 durch eine erste optisch transparente Fläche eines Prismas (110) gebrochen und abgelenkt, durch das Prisma (110) hindurch auf eine verspiegelte Fläche des Prismas (110) geführt und reflektiert und schließlich von einer zweiten transparenten Fläche erneut so gebrochen, dass der auslaufende Referenzstrahl zum einlaufenden entgegengesetzt parallel und seitlich versetzt ist. Es erscheint vorteilhaft, das Prisma (110) symmetrisch auszulegen, wie es in Fig. 2 dargestellt ist. Weiterhin sollten die brechenden Flächen und die reflektierende Fläche eine bauliche Einheit bilden, damit sie eine gemeinsame Bewegung - angedeutet durch Doppelpfeil - entlang der Referenzstrahlrichtung vollführen können ohne die Anordnung zueinander zu ändern.

Allgemein ist es von Vorteil, dass der Referenzstrahl mit einem Prisma und einem Spiegel in einer Ebene wenigstens zweimal gebrochen und genau einmal reflektiert wird. Die Ebene ist dabei die gemeinsame Brechungs- und Reflexionsebene, wobei eine Brechungsebene in Analogie zur Reflexionsebene von der Strahlrichtung und der Normalen der brechenden Fläche aufgespannt wird. Weiterhin wird es als vorteilhaft angesehen, dass ein Prisma (110) mit einer verspiegelnden Beschichtung auf wenigstens einer Seitenfläche verwendet wird.

Es sind jedoch viele alternative Ausgestaltungen möglich, z.B. solche, bei denen kein symmetrisches Prisma benutzt wird. Auch ist es nicht zwingend erforderlich, dass ein verspiegeltes Prisma verwendet wird. Der Spiegel kann z.B. auch in einem vorbestimmten Abstand hinter der rückwärtigen, transparenten Fläche eines Prismas angeordnet werden. In diesem Fall wird der Referenzlichtstrahl das vollständig transparente Prisma an der Rückseite verlassen, einen Luftspalt durchlaufen und dann reflektiert. Auf seinem Weg im Referenzarm wird er dabei insgesamt wenigstens viermal gebrochen und einmal reflektiert.

Apparativ kann ein Luftspalt nachteilig sein, weil an jeder Grenzfläche, die das Licht durchläuft, auch zusätzliche, laufzeitverzögerte Reflexe entstehen, was sich spätestens bei der Interferenz mit dem Probenlicht störend auswirken kann. Doch erzeugt die Transmission des Referenzlichts durch ein brechendes Medium in der Regel auch chromatische Dispersion, die ebenfalls unerwünscht ist. Man wird bestrebt sein, die Weglänge des Referenzlichts durch das brechende Medium möglichst kurz zu halten.

Die Materialauswahl des Prismas und seine Formgebung, d.h. die Anordnung brechender Flächen und des genau einen Spiegels, sind jedenfalls sehr verschieden ausführbar und im Einzelfall - auch unter Beachtung der benutzten Wellenlängen - Gegenstand von Kompromiss- und Optimierungsüberlegungen.

Schließlich ist in Fig. 2 ein fixiert angeordnetes Beugungsgitter (100) als in Transmission Licht ablenkendes Element dargestellt. Im Unterschied zu dem Prisma aus Fig. 1 lenkt das Gitter (100) nicht das gesamte Referenzlicht um einen vorbestimmten Winkel ab, sondern es entstehen ein vom Gitter (100) ausgehendes, divergentes Bündel von Referenzstrahlen zu den Beugungsnebenmaxima und der nicht abgelenkte Strahl zum Hauptmaximum 0. Ordnung. Verwendet man das Gitter (100) dazu, beispielsweise einen Strahl in Richtung auf ein Nebenmaximum 1. Ordnung zum Lichtdetektor (30) zu führen, so muss man in Anbetracht der Strahlbreite darauf achten, dass Strahlen des Bündels zu allen anderen Beugungsordnungen nicht mehr auf den Detektor (30) gelangen können. Dies bedeutet, dass das Gitter (100) in einem Mindestabstand zum Detektor (30) angeordnet werden muss, dessen Betrag leicht geometrisch zu bestimmen ist.

## Patentansprüche

1. Verfahren zur Erzeugung eines zweidimensionalen Interferogramms mit einem Freistrahl-Interferometer des Michelson-Typs umfassend eine ausgedehnte, räumlich teilkohärente Lichtquelle (10) und einen zweidimensionalen Lichtdetektor (30), wobei Licht der Lichtquelle (10) durch einen Strahlteiler (70) mit einem halbdurchlässigen Strahlteilerspiegel in einen Probenlichtstrahl und in einen Referenzlichtstrahl aufgeteilt und einem Probenarm und einem Referenzarm zugeführt wird, wobei der von einer Probe (60) wiederkehrende Probenlichtstrahl über den Strahlteilerspiegel auf den Lichtdetektor geführt wird, wobei der aus dem Referenzarm austretende Referenzlichtstrahl auf dem Lichtdetektor (30) einen vorbestimmten Winkel größer als null mit dem Probenlichtstrahl einschließt, wobei die Länge des Referenzarms veränderlich ist, **dadurch gekennzeichnet, dass** der Referenzlichtstrahl mittels einer ungeraden Anzahl von Reflexionen in jeder Reflexionsebene in wenigstens einem Referenzarmabschnitt zu sich selbst seitlich versetzt und entgegengesetzt parallel verlaufend durch ein am Ausgang des Referenzarmes fixiert angeordnetes, mittels Beugung oder Brechung Licht ablenkendes Element (80, 110) geführt wird.

2. Verfahren nach Anspruch 1, **dadurch gekennzeichnet, dass** die Länge des Referenzarmes in dem wenigstens einen Referenzarmabschnitt geändert wird, in dem der Referenzlichtstrahl zu sich selbst seitlich versetzt und entgegengesetzt parallel verläuft.

3. Verfahren nach Anspruch 2, **dadurch gekennzeichnet, dass** das Referenzlicht hinter dem in der Länge veränderlichen Referenzarmabschnitt durch ein Prisma (80, 110) oder Gitter (100) geführt wird.

4. Verfahren nach einem der vorangehenden Ansprüche, **dadurch gekennzeichnet, dass** der Referenzstrahl über drei Spiegel (90) in einer gemeinsamen Reflexionsebene geführt wird.

5. Verfahren nach einem der Ansprüche 1 bis 3, **dadurch gekennzeichnet, dass** der Referenzstrahl mit einem Prisma (110) und einem Spiegel in einer Ebene wenigstens zweimal gebrochen und genau einmal reflektiert wird.

6. Verfahren nach Anspruch 5, **dadurch gekennzeichnet, dass** ein Prisma (80, 110) mit einer spiegelnden Beschichtung auf wenigstens einer Seitenfläche verwendet wird.

7. Vorrichtung zur Durchführung eines der Verfahren gemäß eines der vorhergehenden Ansprüche, aufweisend ein Freistrahl-Interferometer des Michelson-Typs umfassend eine ausgedehnte, räumlich teilkohärente Lichtquelle (10) und einen zweidimensionalen Lichtdetektor (30) mit einem Strahlteiler (70) und einem halbdurchlässigen Strahlteilerspiegel, wobei Licht der Lichtquelle (10) durch den Strahlteiler (70) mit dem halbdurchlässigen Strahlteilerspiegel in einen Probenlichtstrahl und in einen Referenzlichtstrahl aufgeteilt und einem Probenarm und einem Referenzarm zugeführt wird, wobei der von einer Probe wiederkehrende Probenlichtstrahl über den Strahlteilerspiegel (70) auf den Lichtdetektor (30) geführt wird, wobei der aus dem Referenzarm austretende Referenzlichtstrahl auf dem Lichtdetektor (30) einen vorbestimmten Winkel größer als null mit dem Probenlichtstrahl einschließt, wobei die Länge des Referenzarms veränderlich ist, **dadurch gekennzeichnet, dass** der Aufbau weiterhin ein mittels Beugung oder Brechung Licht ablenkendes Element (80, 110) aufweist, wobei der Referenzlichtstrahl mittels einer ungeraden Anzahl von Reflexionen in jeder Reflexionsebene in wenigstens einem Referenzarmabschnitt zu sich selbst seitlich versetzt und entgegengesetzt parallel verlaufend durch das am Ausgang des Referenzarmes fixiert angeordnete, mittels Beugung oder Brechung Licht ablenkende Element (80, 110) geführt wird.

8. Vorrichtung nach Anspruch 7, **dadurch gekennzeichnet, dass** die Vorrichtung weiterhin über zumindest drei Spiegel (90) verfügt, wobei der Referenzstrahl über die zumindest drei Spiegel (90) in einer gemeinsamen Reflexionsebene geführt wird.

9. Vorrichtung nach Anspruch 7, **dadurch gekennzeichnet, dass** die Vorrichtung weiterhin ein Prisma (80. 110) und einen Spiegel aufweist, wobei der Referenzstrahl mit dem Prisma (80, 110) und dem Spiegel in einer Ebene wenigstens zweimal gebrochen und genau einmal reflektiert wird.

10. Vorrichtung nach Anspruch 9, **dadurch gekennzeichnet, dass** das Prisma (110) eine spiegelnde Beschichtung auf wenigstens einer Seitenfläche aufweist.

## Claims

1. Method for creating a two-dimensional interferogram by a free-beam interferometer of the Michelson-type, comprising an extended, partially spatially coherent light source (10) and a two-dimensional light detector (30), whereby light of the light source (10) is splitted by a beam splitter (70) with a semitransparent beam splitter mirror into a sample light beam and a reference light beam and are directed into a sample arm and a reference arm, whereby the sample light beam returning from a sample (60) is directed by the beam splitter mirror towards the light detector, whereby the reference beam emitting from the reference arm comprises a predefined angle on the light detector (30) of more than 0° with respect to the sample light beam, whereby the length of the reference arm is variable, **characterized in that,** the reference light beam by means of an odd number of reflections within each reflection plane in at least one reference arm section is directed in a manner that it is laterally displaced with respect to itself and travelling in antiparallel direction through a light-deflecting element (80, 110) based on diffraction or refraction affixed to the exit of the reference beam am.

2. Method according to claim 1, **characterized in that** the length of the reference arm in the at least one reference arm section is varied by letting the reference light beam travel laterally displaced with respect to itself and travelling in antiparallel direction.

3. Method according to claim 2, **characterized in that** the reference light beam is directed through a prism (80, 110) or diffraction grating (100) behind the at least one reference arm section being variable in length.

4. Method according to one of the preceding claims, **characterized in that** the reference beam is reflected by three mirrors (90) in a common reflection plane.

5. Method according to one of the preceding claims 1 to 3, **characterized in that** the reference light beam is refracted at least twice and reflected precisely once by a prism (110) and a mirror within a plane.

6. Method according to claim 5, **characterized in that** a prism (80, 110) comprising a reflective coating on at least one lateral surface is used.

7. Device for performing a Method according to one of the preceding claims, comprising a free-beam interferometer of the Michelson-type, comprising an extended, partially spatially coherent light source (10) and a two-dimensional light detector (30), whereby light of the light source (10) is splitted by a beam splitter (70) with a semitransparent beam splitter mirror into a sample light beam and a reference light beam and are directed into a sample arm and a reference arm, whereby the sample light beam returning from a sample is directed by the beam splitter mirror (70) towards the light detector (30), whereby the reference beam emitting from the reference arm comprises a predefined angle on the light detector (30) of more than 0° with respect to the sample light beam, whereby the length of the reference arm is variable, **characterized in that,** the arrangement comprises a light-deflecting element (80, 110), whereby the reference light beam by means of an odd number of reflections within each reflection plane in at least one reference arm section is directed in a manner that it is laterally displaced with respect to itself and travelling in antiparallel directions through a light-deflecting element (80, 110) based on diffraction or refraction affixed to the exit of the reference beam am.

8. Device according to claim 7, **characterized in that** the device comprises at least one reference arm section comprises at least three mirrors (90), wherein the reference light beam is reflected by at least three mirrors (90) within a common reflection plane.

9. Device according to claim 7, **characterized in that** the device comprises a prism (80, 110) and a mirror, wherein the reference light beam is refracted at least twice and reflected precisely once with the prism (80, 110) and the mirror.

10. Device according to claim 9, **characterized in that** the prism (110) has a reflective coating on at least one lateral surface.

## Revendications

1. Procédé, destiné à créer un interférogramme bidimensionnel à l'aide d'un interféromètre à jet libre de type Michelson, comprenant une source lumineuse (10) étendue, partiellement cohérente dans l'espace, et un détecteur de lumière (30) bidimensionnel, de la lumière provenant de la source lumineuse (10) étant divisée par un séparateur de faisceau (70) pourvu d'un miroir séparateur de faisceau semi-transparent en un faisceau lumineux d'échantillonnage et en un faisceau lumineux de référence et étant amenée vers un bras d'échantillonnage et un bras de référence, le faisceau lumineux d'échantillonnage qui revient d'un échantillon (60) étant guidé par l'intermédiaire du miroir séparateur de faisceau sur le détecteur de lumière, le faisceau lumineux de référence qui sort du bras de référence incluant avec le faisceau lumineux d'échantillonnage un angle prédéfini supérieur à zéro sur le détecteur de lumière (30), la longueur du bras de référence étant variable, **caractérisé en ce que** le faisceau lumineux de référence est guidé par diffraction ou réflexion, au moyen d'un nombre impair de réflexions dans chaque plan de réflexion dans au moins un segment de bras de référence, pour s'écouler en étant déporté par rapport à lui-même et à la parallèle dans le sens opposé, à travers un élément (80, 110) de déflexion de lumière par diffraction ou réfraction, placé en étant fixé à la sortie du bras de référence.

2. Procédé selon la revendication 1, **caractérisé en ce que** la longueur du bras de référence est modifiée dans l'au moins un segment de bras de référence, dans lequel le faisceau lumineux de référence s'écoule en étant déporté par rapport à lui-même et à la parallèle dans le sens opposé.

3. Procédé selon la revendication 2, **caractérisé en ce que** la lumière de référence est guidée derrière le segment de référence à longueur variable à travers un prisme (80, 110) ou une grille (100).

4. Procédé selon l'une quelconque des revendications précédentes, **caractérisé en ce que** le faisceau de référence est guidé par l'intermédiaire de trois miroirs (90) dans un plan de réflexion commun.

5. Procédé selon l'une quelconque des revendications 1 à 3, **caractérisé en ce que** le faisceau de référence est réfracté au moins deux fois et réfléchi précisément une fois dans un plan, à l'aide d'un prisme (110) et d'un miroir.

6. Procédé selon la revendication 5, **caractérisé en ce que** l'on utilise un prisme (80, 110) pourvu d'un revêtement réfléchissant sur au moins une surface latérale.

7. Dispositif, destiné à réaliser un procédé selon l'une quelconque des revendications précédentes, comportant un interféromètre à jet libre de type Michelson, comprenant une source lumineuse (10) étendue, partiellement cohérente dans l'espace, et un détecteur de lumière (30) bidimensionnel, avec un séparateur de faisceau (70) et un miroir séparateur de faisceau semi-transparent, de la lumière provenant de la source lumineuse (10) étant divisée par le séparateur de faisceau (70) pourvu du miroir séparateur de faisceau en un faisceau lumineux d'échantillonnage et en un faisceau lumineux de référence et étant amenée vers un bras d'échantillonnage et un bras de référence, le faisceau lumineux d'échantillonnage qui revient d'un échantillon étant guidé par l'intermédiaire du miroir (70) séparateur de faisceau sur le détecteur de lumière (30), le faisceau lumineux de référence qui sort du bras de référence incluant avec le faisceau lumineux d'échantillonnage un angle prédéfini supérieur à zéro sur le détecteur de lumière (30), la longueur du bras de référence étant variable, **caractérisé en ce que** la structure comporte par ailleurs un élément (80,110) déviant la lumière par diffraction ou réfraction, le faisceau lumineux de référence étant guidé au moyen d'un nombre impair de réflexions dans chaque plan de réflexion dans au moins un segment de bras de référence, pour s'écouler en étant déporté par rapport à lui-même et à la parallèle dans le sens opposé, à travers un élément (80, 110) de déflexion de lumière par diffraction ou réfraction, placé en étant fixé à la sortie du bras de référence.

8. Dispositif selon la revendication 7, **caractérisé en ce que** le dispositif dispose par ailleurs d'au moins trois miroirs (90), le faisceau de référence étant guidé par l'intermédiaire des au moins trois miroirs (90) dans un plan de réflexion commun.

9. Dispositif selon la revendication 7, **caractérisé en ce que** le dispositif comporte par ailleurs un prisme (80, 110) et un miroir, le faisceau de référence étant réfracté au moins deux fois et réfléchi précisément une fois dans un plan, à l'aide du prisme (80, 110) et du miroir.

10. Dispositif selon la revendication 9, **caractérisé en ce que** le prisme (110) comporte un revêtement réfléchissant sur au moins une surface latérale.
